# EUROPÄISCHE PATENTSCHRIFT

(11) **EP 2 121 649 B1**
(45) Veröffentlichungstag und Bekanntmachung des Hinweises auf die Patenterteilung: **27.02.2013**
(21) Anmeldenummer: 07856224.6
(22) Anmeldetag: 22.11.2007
(51) Int. Cl.: C07D 307/85

(54) **Verfahren zur Herstellung von Benzofuran-2-carboxamiden**
Method for the production of benzofuran-2-carboxamides
Procédé de production de benzofurane-2-carboxamides

(30) Priorität: 21.12.2006 DE 102006060597
(43) Veröffentlichungstag der Anmeldung: 25.11.2009
(73) Patentinhaber: Merck Patent GmbH, 64293 Darmstadt (DE)
(72) Erfinder: BATHE, Andreas, 64283 Darmstadt (DE)
(86) Internationale Anmeldenummer: PCT/EP2007/010123
(87) Internationale Veröffentlichungsnummer: WO 2008/080456

(56) Entgegenhaltungen:
- WO-A-2005/080336
- DE-A1- 10 112 151
- DE-A1- 19 932 314
- PESCHKE ET AL: "Benzo[b]thiophene-2-carboxamides and benzo[b]furan-2-carboxamides are potent antagonists of the human H3-receptor" BIOORGANIC & MEDICINAL CHEMISTRY LETTERS, OXFORD, GB, Bd. 16, Nr. 12, 15. Juni 2006 (2006-06-15), Seiten 3162-3165, XP005422598 ISSN: 0960-894X
- E. F. DIMAURO ET AL: "Microwave-Assisted Preparation of Fused Bicyclic Heteroaryl Boronates: Application in One-Pot Suzuki Couplings" JOURNAL OF ORGANIC CHEMISTRY, Bd. 71, Nr. 10, 2006, Seiten 3959-3962, XP002475376

## Beschreibung

Die Erfindung betrifft ein Verfahren zur Herstellung von Benzofuran-2-carboxamiden der Formel I 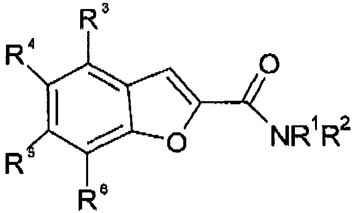 worin
- R¹, R²: H oder unsubstituiertes oder durch A substituiertes Cycloalikyl mit 3 bis 7 C-Atomen oder unverzweigtes oder verzweigtes Alkyl mit 1 bis 10 C- Atomen bedeutet, wobei eine oder mehrere CH₂-Gruppen der Alkylgruppe durch ein O- oder S-Atom, oder durch CH=CH-Gruppen ersetzt sein können oder worin ein oder mehrere Wasserstoffatome der Alkylgruppe durch Hal, OH, Ar, Het, Cycloalkyl mit 3 bis 10 C-Atomen, N(R⁷)₂ CN, COOR⁷, CON(R⁷)₂, NR⁷COR⁷, NR⁷CON(R⁷)₂ NR⁷SO₂A oder SO₂NR⁷ ersetzt sein können
- NR¹R²: zusammen einen drei- bis 7-gliedrigen gesättigten heterocyclischen Ring, wobei zusätzlich 1 oder 2 N- und/oder 1 oder 2 S- und/oder 1 oder 2 O-Atome und/oder eine S(O)ₘ-Gruppe vorliegen können, der durch A, Hal, Cycloalkyl mit 3 bis 10 C-Atomen, OR⁷, N(R⁷)₂, CN, COOR⁷, CON(R⁷)₂ NR⁷COR⁷ und/oder Carbonylsauerstoff substituiert sein kann,
- A: unverzweigtes oder verzweigtes Alkyl mit 1 bis 6 C-Atomen, worin mindestens eine CH₂-Gruppe durch ein O- oder S-Atom, oder durch eine CH=CH-Gruppe ersetzt sein kann, oder mindestens ein H-Atom durch F ersetzt sein kann,
- Ar: unsubstituiertes oder ein- oder mehrfach durch Hal, A, OR⁷, N(R⁷)₂, NO₂, CN, COOR⁷, CON(R⁷)₂, NR⁷COR⁷, NR⁷CON(R⁷)₂, NR⁷SO₂A, COR⁷, SO₂NR⁷ oder S(O)ₘA substituiertes Phenyl, Naphthyl oder Biphenyl, Het einen gesättigten, ungesättigten oder aromatischen mono- oder bicyclischen heterocyclischen Rest mit 5 bis 10 Ringgliedern, wobei 1 bis 4 N- und/oder 1 bis 4 S- und/oder 1 bis 4 O-Atome vorliegen können und der heterocyclische Rest ein-, zwei- oder dreifach durch Hal, A, -[C(R⁷)₂]ₒ-Ar, -[C(R⁷)₂]ₒ-Cycloalkyl, OR⁷, N(R⁷)₂, NO₂, CN, COOR⁷, CON(R⁷)₂, NR⁷COA, NR⁷CON(R⁷)₂, NR⁷SO₂A, COR⁷, SO₂NR⁷ oder S(O)ₘA und/oder Carbonylsauerstoff substituiert sein kann, Hal F, Cl, Br oder I,
- n: 2, 3, 4 oder 5,
- m: 1 oder 2,
- o: 1, 2, 3 oder 4,
- R³, R⁴, R⁵, R⁶: jeweils unabhängig voneinander H, A oder Alkoxy mit 1-20 C-Atomen, Ar, Aryloxy oder COOR₇, Hal, OH, CN, NO₂, N(R⁷)₂, NHCOR⁷, CH₂OH, CH₂OR⁷ oder CO(R⁷)₂, und einer der Reste R³, R⁴, R⁵, R⁶, insbesondere der Rest R⁴, stattdessen auch 4-Benzylpiperazinyl, 4-tert.- Butoxycarbonylpiperazin-1-yl, eine Abgangsgruppe oder 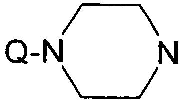
- R⁷: H oder A
- Q eine: Aminoschutzgruppe
bedeuten, durch Umsetzung von Verbindungen der Formel II und III in Gegenwart einer geeigneten Base 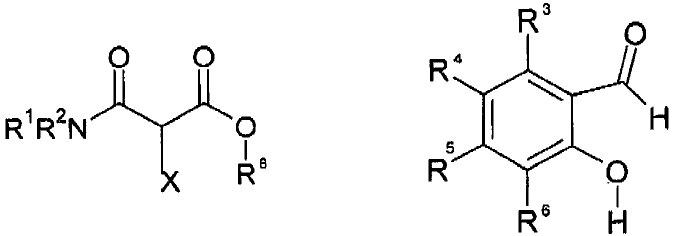 worin
- X: Hal,
- R⁸: A bedeutet und
- R¹-R⁷: die oben angegebene Bedeutung aufweisen.

Die Erfindung ermöglicht insbesondere die Synthese von substituierten Benzofuran-2- Carboxamiden, welche sich als Ausgangsverbindungen in der Herstellung von Arzneimitteln, wie z. B. Antidepressiva eignen. Insbesondere eröffnet sie die Möglichkeit, in einfacher Weise 5-(4-tert.-Butoxycarbonyl-1-piperazinyl)-Benzofuran-2-Carboxamid herzustellen. Verbindungen dieses Typs sind Vorstufen für die Synthese von Antidepressiva, z.B. für das Antidepressivum EMD 68843 (Vilazodon)

Bisher wurden zwei Verfahren zur Herstellung von (I) aus substituierten Salicylaldehyden beschrieben. Dabei wurden substituierte Salicylaldehyde wurden entweder mit XCH₂COOR oder mit XCH(COOR)₂ umgesetzt, wobei R für einen Alkylrest steht. Anschließend erfolgte eine Amidierung. Die bisher bekannten Verfahren sind daher Mehrschritt Verfahren mit relativ geringen Ausbeuten.

Dokumente des allgemeinen Standes der Technik sind Peschke et al. (Bioorganic & Medical Chemistry Letters 2006, 16(12): 3162-3165), Dimauro EF et al. (Journal of Organic Chemistry 2006, 71(10): 3959-3962), WO 2005/080336, DE 101 12 151 und DE 199 32 314.

Der Erfindung lag daher die Aufgabe zugrunde, ein Herstellungsverfahren für Verbindungen der Formel (I) oder deren Salze zu entwickeln, das sowohl eine Vereinfachung darstellt (Einschrittverfahren) als auch eine höhere Ausbeute aufweist.

Es wurde gefunden, dass die Verbindungen der Formel I und ihre Salze, die wichtige Zwischenprodukte zur Herstellung von Arzneimittel darstellen - insbesondere von solchen, die beispielsweise auf das Zentralnervensystem wirken - durch Umsetzung von Verbindungen der Formel II oder deren Salzen mit Verbindungen der Formel III oder deren Salzen erhalten werden können.

Vor- und nachstehend haben die Reste R¹ bis R⁸, sowie Q und X die bei den Formeln I bis III angegebenen Bedeutungen, falls nicht ausdrücklich etwas anderes' angegeben ist.

Der Rest A bedeutet unverzweigtes oder verzweigtes Alkyl und hat 1 bis 6, vorzugsweise 1, 2, 3 oder 4, insbesondere 1 oder 2 C-Atome. Alkyl bedeutet daher insbesondere z.B. Methyl, weiterhin Ethyl, n-Propyl, Isopropyl, n-butyl sek.-Butyl oder tert.-Butyl, ferner auch Pentyl, 1-, 2- oder 3-Methylbutyl, 1,1-, 1,2- oder 2,2- Dimethylpropyl, 1-Ethylpropyl, Hexyl, 1-, 2-, 3- oder 4-Methylpentyl, 1,1-, 1,2-, 1,3-, 2,2-, 2,3- oder 3,3-Dimethylbutyl, 1- oder 2-Ethylbutyl, 1 -Ethyl-1-methylpropyl, 1 -Ethyl-2-methylpropyl, 1,1,2- oder 1,2,2-Trimethylpropyl, worin es möglich ist, dass eine CH₂-Gruppe durch ein 0- oder S-Atom oder durch eine CH=CH-Gruppe ersetzt sein kann oder mindestens ein H-Atom durch F ersetzt sein kann. Der Rest A bedeutet daher ferner beispielsweise Trifluormethyl, Pentafluorethyl, Heptafluorpropyl, Methoxymethyl, Methoxyethyl, Methoxypropyl, Ethoxymethyl, Ethoxyethyl, Ethoxypropyl, Methylsulfanylmethyl, Methylsulfanylethyl, Methylsulfanylpropyl, Ethylsulfanylmethyl, Ethylsulfanylethyl, Ethylsulfanylpropyl, Allyl, Propenyl, But-2-enyl, But-3-enyl, Pent-3-enyl, Pent-4-enyl oder Hex-3-enyl.

Aryl oder Ar bedeutet unsubstituiertes oder ein- oder mehrfach durch Hal, A, OR⁷, N(R⁷)₂, NO₂, CN, COOR⁷, CON(R⁷)₂, NR⁷COR⁷, NR⁷CON(R⁷)₂, NR⁷SO₂A, SO₂NR⁷ oder S(O)ₘA substituiertes Phenyl, Naphthyl oder Biphenyl, wobei A eine der zuvor angegebenen Bedeutungen und R⁷ und m eine der nachfolgend angegebenen Bedeutungen haben.

Ar ist vorzugsweise unsubstituiertes oder substituiertes Phenyl, Naphthyl oder Biphenyl, im einzelnen bevorzugt Phenyl, o-, m- oder p-Tolyl, o-, m- oder p-Ethylphenyl, o-, m- oder p-Propylphenyl, o-, m- oder p-Isopropylphenyl, o-, m- oder p-tert.-Butylphenyl, o-, m- oder p-Trifluormethylphenyl, o-, m- oder p-Hydroxyphenyl, o-, m- oder p-Nitrophenyl, o-, m- oder p-(Trifluormethoxy)-phenyl, o-, m- oder p-Cyanphenyl, o-, m- oder p-Methoxyphenyl, o-, m- oder p-Ethoxyphenyl, o-, m- oder p-Fluorphenyl, o-, m- oder p-Bromphenyl, o-, m- oder p- Chlorphenyl, o-, m- oder p-(Difluormethoxy)-phenyl, o-, m- oder p-(Fluormethoxy)-phenyl, weiter bevorzugt 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2-Chlor-3-methyl-, 2-Chlor- 4-methyl-, 2-Chlor-5-methyl-, 2-Chlor-6-methyl-, 2-Methyl-3-chlor-, 2-Methyl-4-chlor-,2-Methyl-5-chlor-, 2-Methyl-6-chlor-, 3-Chlor-4-methyl-, 3-Chlor-5-methyl- oder 3-Methyl-4-chlorphenyl, 2-Brom-3-methyl-, 2-Brom-4-methyl-, 2-Brom-5-methyl-, 2-Brom-6-methyl-, 2-Methyl-3-brom-, 2-Methyl-4-brom-, 2-Methyl-5-brom-, 2-Methyl-6-brom-, 3-Brom-4-methyl-, 3-Brom-5-methyl- oder 3-Methyl-4-bromphenyl, 2,4-oder 2,5-Dinitrophenyl, 2,5- oder 3,4-Dimethoxyphenyl, 3-Nitro-4-chlorphenyl, 2,3,4-, 2,3,5-, 2,3,6-, 2,4,6- oder 3,4,5-Trichlorphenyl, 2,4,6-tri-tert.-Butylphenyl, ferner bevorzugt 2-Nitro-4-(trifluormethyl)phenyl, 3,5-Di-(trifluormethyl)-phenyl, 2,5-Dimethylphenyl, 2-Hydroxy-3,5-dichlorphenyl, 2-Fluor-5- oder 4-Fluor-3-(trifluormethyl)-phenyl, 4-Chlor-2- oder 4-Chlor-3-(trifluormethyl)-, 2-Chlor-4-oder 2-Chlor-5-(trifluormethyl)-phenyl, 4-Brom-2- oder 4-Brom-3-(trifluormethyl)-phenyl, p-Iodphenyl, 2-Nitro-4-methoxyphenyl, 2,5-Dimethoxy-4-nitrophenyl, 2-Methyl-5-nitrophenyl, 2,4-Dimethyl-3-nitrophenyl, 4-Fluor-3-chlorphenyl, 4-Fluor-3,5-dimethylphenyl, 2-Fluor-4-Bromphenyl, 2,5-Difluor-4-bromphenyl, 2,4-Dichlor-5-methylphenyl, 3-Brom-6-methoxyphenyl, 3-Chlor-6-methoxyphenyl, 2-Methoxy-5-methylphenyl oder 2,4,6-Triisopropylphenyl. Besonders bevorzugt ist Ar Phenyl.

Unsubstituiertes oder durch A substituiertes Cycloalkyl mit 3 bis 10 C-Atomen bedeutet vorzugsweise Cyclopropyl, Cyclobutyl, Cyclopentyl, Cyclohexyl, 4-Methylcyclohexyl, Cycloheptyl oder Cyclooctyl. Cycloalkyl bedeutet ebenfalls mono- oder bicyclische Terpene, vorzugsweise p-Menthan, Menthol, Pinan, Bornan oder Campher, wobei jede bekannte stereoisomere Form eingeschlossen ist oder Adamantyl. Für Campher bedeutet dies sowohl L-Campher als auch D-Campher. Besonders bevorzugt ist Cycloalkyl Cyclopropyl, Cyclobutyl, Cyclopentyl, Cyclohexyl, Cycloheptyl und 4-Methylcyclohexyl.

Hal bedeutet Fluor, Chlor, Brom oder Iod, insbesondere Chlor oder Brom. In Verbindungen der Formel I bedeutet Hal besonders bevorzugt Fluor.

Het bedeutet einen gesättigten, ungesättigten oder aromatischen mono- oder bicyclischen heterocyclischen Rest mit 5 bis 10 Ringgliedern, wobei 1 bis 4 N und/oder 1 bis 4 S- und/oder 1 bis 4 O-Atome vorliegen können und der heterocyclische Rest ein-, zwei- oder dreifach durch Hal. A, -[C(R⁷)₂]ₒ-Ar, - [C(R⁷)₂]ₒ-Cycloalkyl. OR⁷, N(R⁷)₂, NO₂, CN, COOR⁷, CON(R⁷)₂, NR⁷COA, NR⁷CON(R⁷)₂, NR⁷SO₂A, COR⁷, SO₂NR⁷ oder S(O)ₘA und/oder Carbonylsauerstoff substituiert sein kann, wobei A und Cycloalkyl eine der zuvor angegebenen Bedeutungen haben und R⁷, m und o eine der nachstehend angegebenen Bedeutungen haben.

Het ist vorzugsweise substituiertes oder unsubstituiertes 2- oder 3-Furyl, 2- oder 3-Thienyl, 1-,2- oder 3-Pyrrolyl, 1-, 2-, 4- oder 5-Imidazolyl, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-I -1-, -4- oder-5-yl, 1,2,4-Triazol-1 -, -4 oder - 5-yl, 1 - oder 5-Tetrazolyl, 1,2,3-Oxadiazol-4- oder -5-yl 1,2,4-Oxadiazol-3- oder -5-yl, 1,3,4-Thiadiazol-2- oder-5-yl, 1,2,4-Thiadiazol-3- oder-5-yl, 1,2,3-Thiadiazol-4- oder -5-yl, 2-, 3-, 4-, 5- oder 6- 2H-Thiopyranyl, 2-, 3- oder 4-4H-Thiopyranyl, 3- oder 4-Pyridazinyl, Pyrazinyl, 2-, 3-, 4-, 5-, 6- oder 7-Benzofuryl, 2-, 3-, 4-, 5-, 6- oder 7-Benzothienyl, I-, 2-, 3-, 4-, 5-, 6- oder 7-1H-Indolyl, 1-, 2-, 4- oder 5-Benzimidazolyl, 1-, 3-, 4-, 5-, 6- oder 7-Benzopyrazolyl, 2-, 4-, 5-, 6-oder 7-Benzoxazolyl, 3-, 4-, 5-, 6- oder 7-Benzisoxazolyl, 2-, 4-, 5-, 6- oder 7-Benzthiazolyl, 4- oder 5-Benzothiadiazolyl, 2-, 4-, 5-, 6- oder 7-Benzisothiazolyl, 4-, 5-, 6- oder 7-Benz-2,1,3-oxadiazolyl, 1-, 2-, 3-, 4-, 5-, 6-, 7- oder 8-Chinolinyl, 1-, 3-, 4-, 5-, 6-, 7- oder 8-Isochinolinyl, 1-, 2-, 3-, 4- oder 9-Carbazolyl, 1-, 2-, 3-, 4-, 5-, 6-, 7-, 8- oder 9-Acridinyl, 3-, 4-, 5-, 6-, 7- oder 8- Cinnolinyl, 2-, 4-, 5-, 6-, 7- oder 8-Chinazolinyl. Die heterocyclischen Reste können auch teilweise oder vollständig hydriert sein. Het kann also auch bedeuten 2,3-Dihydro-2-, -3-, -4-oder -5-furyl, 2,5-Dihydro-2-, -3-, -4- oder -5-furyl, Tetrahydro-2- oder -3-furyl, 1,3-Dioxolan-4-yl, Tetrahydro-2- oder -3-thienyl, 2,3-Dihydro-1 -, -2-, -3-, -4-oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -3-pyrollyl,Tetrahydro-1-, -2- oder 4-imidazolyl, 2,3-Dihydro-1-, -2-, -3-, -4-, -5-, -6-, -7-1H-indolyl, 2,3-Dihydro-1-, -2-, -3-, -4- oder -5-pyrazolyl, Tetrahydro-1-, -3- oder -4-pyrazolyl, 1,4-Dihydro-1-, -2-, -3- oder -4-pyridyl, 1,2,3,4-Tetrahydro-1-, -2-, -3-, -4-, -5- oder-6-pyridyl, 1,2,3,6-Tetrahydro-1-, -2-, -3, -4-, -5- oder -6-pyridyl, 1-, 2-, 3- oder 4-Piperidinyl, 1-, 2-, 3- oder 4-Azepanyl, 2-, 3- oder 4-Morpholinyl, Tetrahydro-2-, -3- oder-4-pyranyl, 1,4-Dioxanyl, 1,3-Dioxan-2-, -4- oder -5-yl, Hexahydro-1-, -3- oder -4-pyridazinyl, Hexahydro-1-, -2-, -4- oder -5-pyrimidinyl, 1-, 2- oder 3- Piperazinyl, 1,2,3,4-Tetrahydro-1 -, -2-, -3-, -4-, -5-, -6-, -7- oder -8-chinolinyl, 1,2,3,4-Tetrahydro-1-, -2-, -3-, -4-, -5-, -6-, -7- oder -8-isochinolinyl.

Het ist besonders bevorzugt unsubstituiertes Furan-2-yl, Tetrahydrofuran-2-yl, Pyridin-4-yl, Pyridin-3-yl, Pyridin-2-yl, Thiophen-2-yl oder Imidazol-5-yl.

R¹ bedeutet unsubstituiertes oder durch A substituiertes Cycloalkyl mit 3 bis 10 C-Atomen oder unverzweigtes oder verzweigtes Alkyl mit 1 bis 10 C-Atomen, worin eine oder mehrere CH₂-Gruppen der Alkylgruppe durch ein O- oder S-Atom, durch CH=CH-Gruppen oder durch C=C-Gruppen ersetzt sein können oder worin ein odermehrere Wasserstoffatome der Alkylgruppe durch Hal, OH, Ar, Het, Cycloalkyl mit 3 bis 10 C-Atomen, N(R⁷)₂, CN, COOR⁷, CON(R⁷)₂, NR⁷COR⁷, NR⁷CON(R⁷)₂, NR⁷SO₂A oder SO₂NR⁷ ersetzt sein können, wobei A, Ar, Hal, Het und Cycloalkyl eine der vorstehend angegebenen Bedeutungen haben und R⁷ eine der nachstehend angegebenen Bedeutungen hat.

R¹ bedeutet weiterhin bevorzugt H, Allyl, Benzyl, Phenylethyl, 2-Methoxyethyl, 3-Methoxypropyl, 3-Ethoxypropyl, Aminocarbonylmethyl, 2-Dimethylaminoethyl, 2-Diethylaminoethyl, 3-Dimethylaminopropyl, 4-Dimethylaminobutyl, 2-Methylaminoethyl, Cyanmethyl, 2,2,2-Trifluorethyl, 2-Hydroxyethyl, 3-Hydroxypropyl, Prop-2-inyl, Cyclopropyl, Cyclobutyl, Cyclopentyl, Cyclohexyl, Cycloheptyl, 4-Methyl-cyclohexyl, Cyclohexyl-methyl, Furan-2-yl-methyl, 2-Morpholin-4-yl-ethyl, Pyridin-3-ylmethyl, Pyridin-2-ylmethyl, Pyridin-4-ylmethyl, 2-Imidazol-5-ylethyl, Thiophen-2-ylmethyl oder Tetrahydrofuran-2-ylmethyl.

R² bedeutet H, A oder R¹, wobei A und R¹ eine der zuvor angegebenen Bedeutungen haben. Besonders bevorzugt ist R² H.

NR¹R² bedeutet zusammen einen drei- bis 7-gliedrigen gesättigten heterocyclischen Ring, wobei zusätzlich 1 oder 2 N- und/oder 1 oder 2 S-und/oder 1 oder 2 O-Atome und/oder eine S(O)ₘ-Gruppe vorliegen können, der durch A, Hal, Cycloalkyl mit 3 bis 10 C-Atomen, OR⁷, N(R⁷)₂, CN, COOR⁷, CON(R⁷)₂, NR⁷COR⁷ und/oder Carbonylsauerstoff substituiert sein kann, wobei A, Hal und Cycloalkyl eine der zuvor angegebenen Bedeutungen haben und R⁷ und m eine der nachstehend genannten Bedeutungen haben.

NR¹R² ist vorzugsweise 1-, 2- oder 3-Pyrrolidinyl, Tetrahydro-1-, -2- oder 4-imidazolyl, Tetrahydro-1-, -3- oder-4-pyrazolyl, 1-, 2,- 3- oder 4-Piperidinyl, 1-, 2,-, 3- oder 4-Azepanyl, 2-, 3- oder 4-Morpholinyl, 1,4 -Dioxanyl, 1,3-Dioxan-2-, - 4- oder -5-yl, Hexahydro-1-, -3- oder -4-pyridazinyl, Hexahydro-1-, -2-, -4- oder - 5-pyrimidinyl oder 1-, 2- oder 3- Piperazinyl. NR¹R² ist besonders bevorzugt Piperidin-1-yl und Morpholin-1-yl.

n bedeutet 2, 3, 4 oder 5, besonders bevorzugt 4.
m bedeutet 1 oder 2.
o bedeutet 0, 1, 2, 3 oder 4.

Abgangsgruppe bedeutet eine bei der Reaktion austretende Gruppe. Insbesondere sind hierfür die Gruppen Hal, wie Cl oder Br, Toluolsulfonyl, Benzolsulfonyl, Methansulfonyl, und Trifluormethansulfonyl geeignet.

Die Bezeichnung Aminoschutzgruppe bezieht sich auf Gruppen, die in der Lage sind, Aminogruppen vor chemischen Reaktionen zu schützen, die sich aber nach Ablauf der gewünschten Reaktion - an anderer Stelle im Molekül - leicht entfernen lassen. Typische Aminoschutzgruppen sind insbesondere unsubstituierte Acyl-, Aryl-, Aralkoxymethyl- oder Aralkyl-Gruppen.
Da die Aminoschutzgruppen nach der gewünschten Reaktion entfernt werden, spielen Typ und Größe eine untergeordnete Rolle. Dennoch sind Aminoschutzgruppen mit 1 bis 20, vorzugsweise 1 bis 8 C-Atomen besonders bevorzugt. Unter Acyl-Gruppen werden Derivate von aliphatischen, araliphatischen, aromatischen oder heterocyclischen Carbonsäuren oder Sulfonsäuren und insbesondere Alkoxycarbonyl-, Aryloxycarbonyl- und ganz besonders Aralkoxycarbonyl-Gruppen verstanden. Beispiele für Acylgruppen dieses Typs sind Alkanoyl, wie Acetyl, Propionyl, Butyryl; Aralkanoyl, wie Phenylacetyl; Aroyl, wie Benzoyl oder Tolyl; Aryloxyalkanoyl, wie Phenoxyacetyl; Alkoxycarbonyl, wie Methoxycarbonyl, Ethoxycarbonyl, 2,2,2-Trichloroethoxycarbonyl, BOC (tert-Butoxycarbonyl), 2-lodethoxycarbonyl; Aralkyloxycarbonyl, wie CBZ (Carbobenzoxycarbonyl), 4-Methoxybenzyloxycarbonyl, FMOC (9-Fluorenylmethoxycarbonyl); Arylsulfonyl, wie Mtr (4-Methoxy-2,3,6-trimethylphenylsulfonyl).
Insbesondere bedeutet Aminoschutzgruppe Benzyl oder BOC.

R³, R⁴, R⁵ und R⁶ bedeuten unabhängig voneinander vorzugsweise H, Methyl oder Ethyl, insbesondere aber H.

R⁴ bedeutet insbesondere bevorzugt Methyl, Ethyl, 4-Benzylpiperazinyl oder 4-tert-Butoxycarbonylpiperazin-1-yl oder eine Abgangsgruppe, insbesondere aber H oder 4-tert.- Butoxycarbonylpiperazin-1 -yl oder eine Abgangsgruppe.

R⁷ und R⁸ bedeuten unabhängig voneinander vorzugsweise Methyl oder Ethyl, insbesondere aber Ethyl.

Eine besonders bevorzugte Verbindung der Formel II ist
Brom-malonsäuremonoethylester-monoamid.

Besonders bevorzugte Verbindungen der Formel III sind 5-(4-tert.-Butoxycarbonylpiperazin-1 -yl)-2-hydroxybenzaldehyd oder

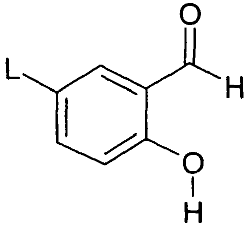

Worin L eine Abgangsgruppe und bevorzugt Br bedeutet. Im erfindungsgemäßen Verfahren wird die Verbindung der Formel III vorzugsweise in einem Lösungsmittel, wie z. B. Wasser, Alkohol,/Ether, gesättigte oder aromatische halogenierte oder halogenfreie Kohlenwasserstoffe oder deren Mischungen, insbesondere aber in polaren aprotischen Lösungsmittel wie z.B. Dimethylformamid, gelöst oder suspendiert. Eine geeignete Base wie z. B. Kaliumcarbonat oder Alkalialkoholat wird zugegeben. Anschließend wird die Verbindung der Formel II hinzugegeben und für 1 bis 12 h, vorzugsweise 1,5 bis 8 h gerührt. Für weitere 1 bis 24 h, vorzugsweise für 2 bis 10 h wird das Reaktionsgemisch auf 20 °C bis 200 °C, vorzugsweise auf 50 °C bis 180 °C und insbesondere auf 80 °C bis 150 °C erwärmt. Die so entstandene Zielverbindung wird durch übliche Aufarbeitung erhalten.

Übliche Aufarbeitung bedeutet vorzugsweise Filtration, Zugabe von Wasser und erneute Filtration.

Im folgenden werden bevorzugte Ausführungsformen des erfindungsgemäßen Verfahrens genannt:
Verfahren zur Herstellung von Verbindungen der Formel I, dadurch gekennzeichnet, dass die Umsetzung der Verbindungen der Formeln II mit den Verbindungen der Formel III zu Verbindungen der Formel I bei 0°C-200°C erfolgt.

Verfahren zur Herstellung von Verbindungen der Formel I, dadurch gekennzeichnet, dass die Umsetzung der Verbindungen der Formeln II mit den Verbindungen der Formel III zu Verbindungen der Formel I bei pH 7 bis 14 erfolgt, vorzugsweise bei pH 8-11 erfolgt.

Die mit Hilfe des erfindungsgemäßen Verfahrens hergestellten Substanzen können als Vorstufen für die Synthese von Antidepressiva, insbesondere für das Antidepressivum EMD 68843 (Vilazodon) dienen.
Im folgenden bedeutet °C Grad Celsius.

### Beispiel 1:

1.4 g 2-Hydroxybenzaldehyd werden unter Rühren in 15 ml Dimethylformamid bei 20 °C gelöst. 2,1 g Kaliumcarbonat werden zugegeben und für 15 min. gerührt. Anschließend werden 2,8 g 2-Brom-malonsäure-monoethylestermonoamid zudosiert. Die Mischung wird für 2 Std. bei Raumtemperatur gerührt, dann für 5 Std. bei 120 °C gerührt. Nach Abkühlung auf 20 °C wird der Feststoffanteil abfiltriert; der Filterrückstand wird mit 20 ml Dimethylformamid gewaschen und verworfen. Die vereinigten Filtrate werden unter Rühren langsam mit 150 ml Wasser verdünnt und für 1 Std. bei 20 °C gerührt. Das angefallene, feste Produkt wird abfiltriert, der Filterrückstand mit Wasser gewaschen und getrocknet. Der anfallende Feststoff wird aus Toluol/n-Heptan umkristallisiert.

### Beispiel 2:

3.5 g 5-(4-tert.-Butoxycarbonylpiperazin-1-yl)-2-Hydroxybenzaldehyd werden unter Rühren in 15 ml Dimethylformamid bei 20 °C gelost. 2,1 g Kaliumcarbonat werden zugegeben und für 15 min gerührt. Anschließend werden 2,8 g 2-Brom-Malonsäure-Monoethylester-Monoamid zudosiert. Die Mischung wird für 2 Std. bei Raumtemperatur gerührt, dann für 5 Std. bei 120 °C gerührt. Nach Abkühlung auf 20 °C wird der Feststoffanteil abfiltriert; der Filterrückstand wird mit 20 ml Dimethylformamid gewaschen und verworfen. Die vereinigten Filtrate werden unter Rühren langsam mit 150 ml Wasser verdünnt und für 1 Std. bei 20 °C gerührt. Das angefallene, feste Produkt wird abfiltriert, der Filterruckstand mit Wasser gewaschen und getrocknet. Der anfallende Feststoff wird aus Toluol/n-Heptan umkristallisiert.

## Patentansprüche

1. Verfahren zur Herstellung von Benzofuran-2-carboxamiden der Formel I 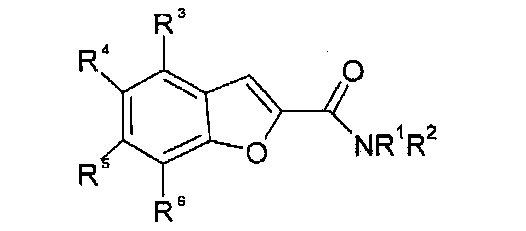 worin
R¹, R² H oder unsubstituiertes oder durch A substituiertes Cycloalkyl mit 3 bis 7 C-Atomen oder unverzweigtes oder verzweigtes Alkyl mit 1 bis 10 C-Atomen bedeutet, wobei eine oder mehrere CH₂-Gruppen der Alkylgruppe durch ein O- oder S-Atom oder durch CH-CH-Gruppen ersetzt sein können oder worin ein oder mehrere Wasserstoffatome der Alkylgruppe durch Hal, OH, Ar, Het, Cycloalkyl mit 3 bis 10 C-Atomen, N(R⁷)₂ CN , COOR⁷, CON(R⁷)₂, NR⁷COR⁷, NR⁷CON(R⁷)₂, NR⁷SO₂A oder SO₂NR⁷ ersetzt sein können,
NR¹R² zusammen einen drei- bis 7-gliedrigen gesättigten heterocyclischen Ring, wobei zusätzlich 1 oder 2 N- und/oder 1 oder 2 S- und/oder 1 oder 2 O-Atome und/oder eine S(O)ₘ-Gruppe vorliegen können, der durch A, Hal, Cycloalkyl mit 3 bis 10 C-Atomen, OR⁷, N(R⁷)₂, CN, COOR⁷, CON(R⁷)₂, NR⁷COR⁷ und/oder Carbonylsauerstoff substituiert sein kann,
A unverzweigtes oder verzweigtes Alkyl mit 1 bis 6 C-Atomen, worin mindestens eine CH₂-Gruppe durch ein O- oder S-Atom, oder durch eine CH=CH-Gruppe ersetzt sein kann, oder mindestens ein H-Atom durch F ersetzt sein kann,
Ar unsubstituiertes oder ein- oder mehrfach durch Hal, A, OR⁷, N(R⁷)₂, NO₂, CN, COOR⁷,CON(R⁷)₂, NR⁷COR⁷, NR⁷CON(R⁷)₂, NR⁷SO₂A, COR⁷, SO₂NR⁷ oder S(O)ₘA substituiertes Phenyl, Naphthyl oder Biphenyl,
Het einen gesättigten, ungesättigten oder aromatischen mono- oder bicyclischen heterocyclischen Rest mit 5 bis 10 Ringgliedern, wobei 1 bis 4 N- und/oder 1 bis 4 S- und/oder 1 bis 4 O-Atome vorliegen können und der heterocyclische Rest ein-, zwei- oder dreifach durch Hal, A, -[C(R⁷)₂]ₒ-Ar, -[C(R⁷)₂]ₒ-Cycloalkyl, OR⁷, N(R⁷)₂, NO₂, CN, COOR⁷, CON(R⁷)₂, NR⁷COA, NR⁷CON(R⁷)₂, NR⁷SO₂A, COR⁷, SO₂NR⁷ oder S(O)ₘA und/oder Carbonylsauerstoff substituiert sein kann,
Hal F, Cl, Br oder I,
n 2, 3, 4 oder 5,
m 1 oder 2,
o 0, 1, 2, 3 oder 4,
R³, R⁴, R⁵, R⁶ jeweils unabhängig voneinander H, A oder Alkoxy mit 1-20 C-Atomen, Ar, Aryloxy oder COOR⁷, Hal, OH, CN, NO₂, N(R⁷)₂, NHCOR⁷, CH₂OH, CH₂OR⁷ oder CO(R⁷)₂, und einer der Reste R³, R⁴, R⁵, R⁶, stattdessen auch 4-Benzyl-piperazinyl, 4-tert.-Butoxycarbonylpiperazin-1-yl, eine Abgangsgruppe oder 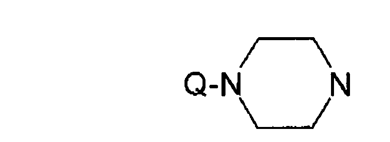
R⁷ H oder A
Q eine Aminoschutzgruppe
bedeuten, durch Umsetzung von Verbindungen der Formel II und III in Gegenwart einer geeigneten Base 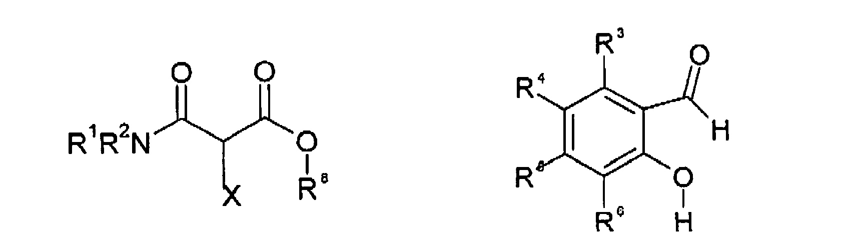 worin
X Hal,
R⁸ A bedeutet und
R¹-R⁷ die oben angegebene Bedeutung aufweisen.

2. Verfahren nach Anspruch 1, worin R⁴ eine Abgangsgruppe, 4-tert.-Butoxycarbonyl- piperazin-1-yl oder 4-Benzylpiperazinyl bedeutet.

3. Verfahren nach einem oder mehreren der Ansprüche 1 bis 2, worin R³, R⁵ und R⁶ unabhängig voneinander H oder Methyl bedeuten.

4. Verfahren nach einem oder mehreren der Ansprüche 1 bis 3, worin R⁷ H bedeutet.

5. Verfahren nach einem oder mehreren der Ansprüche 1 bis 4, worin R⁸ Alkyl bedeutet.

6. Verfahren zur Herstellung von 5-(4-tert.-Butoxycarbonyl-1-piperazinyl)-Benzofuran-2-Carboxamid nach einem oder mehreren der Ansprüche 1 bis 5.

7. Verfahren nach einem der vorhergehenden Ansprüche, **dadurch gekennzeichnet, dass** die Umsetzung der Verbindungen der Formel II mit den Verbindungen der Formel III in einem polaren aprotischen Lösungsmittel stattfindet.

8. Verfahren zur Herstellung von Verbindungen der Formel I nach einem oder mehreren der Ansprüche 1 bis 7, **dadurch gekennzeichnet, dass** die Umsetzung der Verbindungen der Formel II mit den Verbindungen der Formel III zu Verbindungen der Formel I bei pH-Werten zwischen 7 und 14 und bei Temperaturen von 0 - 200 °C erfolgt.

9. Verfahren nach einem der vorhergehenden Ansprüche, **dadurch gekennzeichnet, dass** als Verbindung der Formel III 5-(4-tert.-Butoxycarbonylpiperazin-1-yl)-2-hydroxybenzaldehyd eingesetzt wird.

## Claims

1. Process for the preparation of benzofuran-2-carboxamides of the formula I 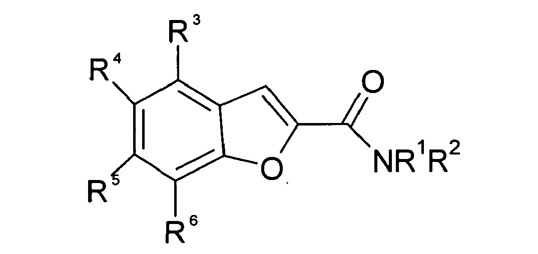 in which
R¹, R² denote H or cycloalkyl having 3 to 7 C atoms or unbranched or branched alkyl having 1 to 10 C atoms, each of which is unsubstituted or substituted by A, where one or more CH₂ groups of the alkyl group may be replaced by an O or S atom or by CH=CH groups or in which one or more hydrogen atoms of the alkyl group may be replaced by Hal, OH, Ar, Het, cycloalkyl having 3 to 10 C atoms, N(R⁷)₂, CN , COOR⁷, CON(R⁷)₂, NR⁷COR⁷, NR⁷CON(R⁷)₂, NR⁷SO₂A or SO₂NR⁷,
NR¹R² together denotes a three- to 7-membered saturated heterocyclic ring, in which, in addition, 1 or 2 N and/or 1 or 2 S and/or 1 or 2 O atoms and/or one S(O)ₘ group may be present and which may be substituted by A, Hal, cycloalkyl having 3 to 10 C atoms, OR⁷, N(R⁷)₂, CN, COOR⁷, CON(R⁷)₂, NR⁷COR⁷ and/or carbonyl oxygen,
A denotes unbranched or branched alkyl having 1 to 6 C atoms, in which at least one CH₂ group may be replaced by an O or S atom, or by a CH=CH group, or at least one H atom may be replaced by F,
Ar denotes phenyl, naphthyl or biphenyl, each of which is unsubstituted or mono- or polysubstituted by Hal, A, OR⁷, N(R⁷)₂, NO₂, CN, COOR⁷, CON(R⁷)₂, NR⁷COR⁷, NR⁷CON(R⁷)₂, NR⁷SO₂A, COR⁷, SO₂NR⁷ or S(O)ₘA,
Het denotes a saturated, unsaturated or aromatic mono- or bicyclic heterocyclic radical having 5 to 10 ring members, where 1 to 4 N and/or 1 to 4 S and/or 1 to 4 O atoms may be present and the heterocyclic radical may be mono-, di- or trisubstituted by Hal, A, -[C(R)₂]ₒ-Ar, -[C(R⁷)₂]ₒ-cycloalkyl, OR⁷, N(R⁷)₂, NO₂, CN, COOR⁷, CON(R⁷)₂, NR⁷COA, NR⁷CON(R⁷)₂, NR⁷SO₂A, COR⁷, SO₂NR⁷ or S(O)ₘA and/or carbonyl oxygen,
Hal denotes F, Cl, Br or I,
n denotes 2, 3, 4 or 5,
m denotes 1 or 2,
o denotes 0, 1, 2, 3 or 4,
R³, R⁴, R ⁵ R⁶ each, independently of one another, denote H, A or alkoxy having 1-20 C atoms, Ar, aryloxy or COOR₇, Hal, OH, CN, NO₂, N(R⁷)₂, NHCOR⁷, CH₂OH, CH₂OR⁷ or CO(R⁷)₂, and one of the radicals R³, R⁴, R⁵, R⁶ instead also denotes 4-benzylpiperazinyl, 4-tert-butoxycarbonylpiperazin-1-yl, a leaving group or 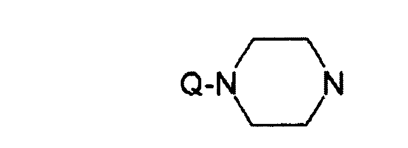
R⁷ denotes H or A
Q denotes an amino-protecting group,
by reaction of compounds of the formulae II and III in the presence of a suitable base 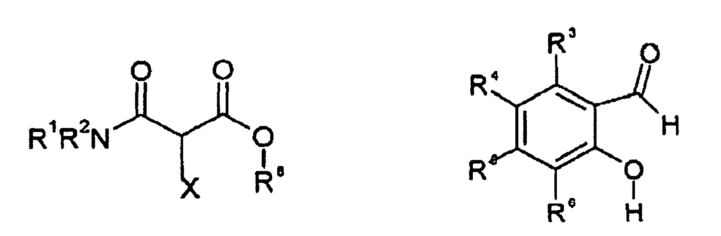 in which
X denotes Hal,
R⁸ denotes A and
R⁷-R⁷ have the meaning indicated above.

2. Process according to Claim 1, in which R⁴ denotes a leaving group, 4-tert-butoxycarbonylpiperazin-1-yl or 4-benzylpiperazinyl.

3. Process according to one or more of Claims 1 to 2, in which R³, R⁵ and R⁶, independently of one another, denote H or methyl.

4. Process according to one or more of Claims 1 to 3, in which R⁷ denotes H.

5. Process according to one or more of Claims 1 to 4, in which R⁸ denotes alkyl.

6. Process for the preparation of 5-(4-tert-butoxycarbonyl-1-piperazinyl)benzofuran-2-carboxamide according to one or more of Claims 1 to 5.

7. Process according to one of the preceding claims, **characterised in that** the reaction of the compounds of the formula II with the compounds of the formula III is carried out in a polar aprotic solvent.

8. Process for the preparation of compounds of the formula I according to one or more of Claims 1 to 8, **characterised in that** the reaction of the compounds of the formulae II with the compounds of the formula III to give compounds of the formula I is carried out at pH values between 7 and 14 and at temperatures of 0 - 200°C.

9. Process according to one of the preceding claims, **characterised in that** the compound of the formula III employed is 5-(4-tert-butoxycarbonylpiperazin-1 -yl)-2-hydroxybenzaldehyde.

## Revendications

1. Procédé pour la préparation de benzofuran-2-carboxamides de la formule I 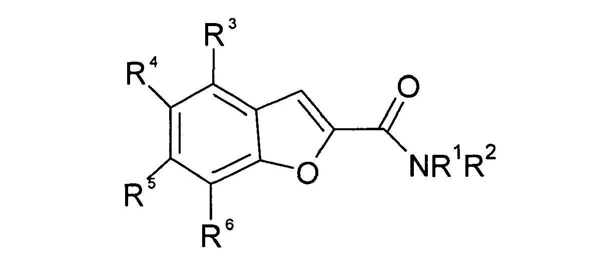 dans laquelle
R¹, R² représentent H ou cycloalkyle comportant 3 à 7 atomes de C ou alkyle non ramifié ou ramifié comportant 1 à 10 atomes de C, dont chacun est non substitué ou substitué par A, où un ou plusieurs groupes CH₂ du groupe alkyle peut/peuvent être remplacé(s) par un atome de O ou de S ou par des groupes CH=CH ou où un ou plusieurs atomes d'hydrogène du groupe alkyle peut/peuvent être remplacé(s) par Hal, OH, Ar, Het, cycloalkyle comportant 3 à 10 atomes de C, N(R⁷)₂, CN , COOR⁷, CON(R⁷)₂, NR⁷COR⁷, NR⁷CON(R⁷)₂, NR⁷SO₂A ou SO₂NR⁷_{,}
NR¹R² en association représente un cycle hétérocyclique saturé de trois à sept éléments, où, en outre, 1 ou 2 atomes de N et/ou 1 ou 2 atomes de S et/ou 1 ou 2 atomes de O et/ou un seul groupe S(O)ₘ peut/peuvent être présent(s) et lequel peut être substitué par A, Hal, cycloalkyle comportant de 3 à 10 atomes de C, OR⁷, N(R⁷)₂, CN, COOR⁷, CON(R⁷)₂, NR⁷COR⁷ et/ou oxygène carbonyle,
A représente alkyle non ramifié ou ramifié comportant de 1 à 6 atomes de C, où au moins un groupe CH₂ peut être remplacé par Ar un atome de O ou de S, ou par un groupe CH=CH, ou au moins un atome de H peut être remplacé par F, représente phényle, naphtyle ou biphényle dont chacun est non substitué ou mono- ou polysubstitué par Hal, A, OR⁷, N(R⁷)₂, NO₂, CN, COOR⁷, CON(R⁷)₂, NR⁷COR⁷, NR⁷CON(R⁷)₂, NR⁷SO₂A, COR⁷, SO₂NR⁷ ou S(O)ₘA,
Het représente un radical hétérocyclique mono- ou bicyclique saturé, non saturé ou aromatique comportant 5 à 10 éléments de cycle, où 1 à 4 atomes de N et/ou 1 à 4 atomes de S et/ou 1 à 4 atomes de O peut/peuvent être présent(s) et le radical hétérocyclique peut être mono-, di- ou trisubstitué par Hal, A, -[C(R⁷)₂]ₒ-Ar, -[C(R⁷)₂]ₒ-cycloalkyle, OR⁷, N(R⁷)₂, NO₂, CN, COOR⁷, CON(R⁷)₂, NR⁷COA, NR⁷CON(R⁷)₂, NR⁷SO₂A, COR⁷, SO₂NR⁷ ou S(O)ₘA et/ou oxygène carbonyle,
Hal représente F, CI, Br ou I,
n représente 2, 3, 4 ou 5,
m représente 1 ou 2,
o représente 0, 1, 2, 3 ou 4,
R³, R⁴, R⁵, R⁶ représentent, chacun indépendamment des autres, H, A ou alcoxy comportant 1-20 atomes de C, Ar, aryloxy ou COOR₇, Hal, OH, CN, NO₂, N(R⁷)₂, NHCOR⁷, CH₂OH, CH₂OR⁷ ou CO(R⁷)₂, et l'un des radicaux R³, R⁴, R⁵, R⁶ représente également en lieu et place 4-benzylpipérazinyle, 4-tert-butoxycarbonylpipérazine-1-yle, un groupe partant ou 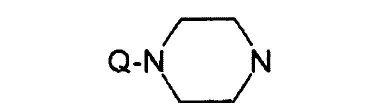
R⁷ représente H ou A,
Q représente un groupe d'amino-protection,
par réaction de composés des formules II et III en présence d'une base appropriée 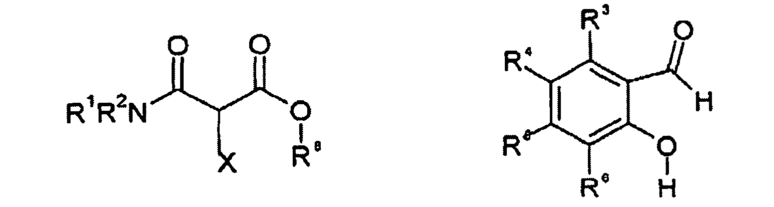 dans lesquelles
X représente Hal,
R⁸ représente A et
R¹-R⁷ présentent la signification indiquée ci avant.

2. Procédé selon la revendication 1, dans lequel R⁴ représente un groupe partant, 4-tert-butoxycarbonylpipérazine-1-yle ou 4-benzylpipérazinyle.

3. Procédé selon une ou plusieurs des revendications 1 à 2, dans lequel R³, R⁵ et R⁶, indépendamment les uns des autres, représentent H ou méthyle.

4. Procédé selon une ou plusieurs des revendications 1 à 3, dans lequel R⁷ représente H.

5. Procédé selon une ou plusieurs des revendications 1 à 4, dans lequel R⁸ représente alkyle.

6. Procédé pour la préparation de 5-(4-tert-butoxycarbonyl-1-pipérazinyl)benzofuran-2-carboxamide selon une ou plusieurs des revendications 1 à 5.

7. Procédé selon l'une des revendications précédentes, **caractérisé en ce que** la réaction des composés de la formule II avec les composés de la formule III est mise en oeuvre dans un solvant aprotique polaire.

8. Procédé pour la préparation de composés de la formule I selon une ou plusieurs des revendications 1 à 8, **caractérisé en ce que** la réaction des composés de la formule II avec les composés de la formule III pour obtenir des composés de la formule I est mise en oeuvre à des valeurs de pH entre 7 et 14 et à des températures de 0 - 200°C.

9. Procédé selon l'une des revendications précédentes, **caractérisé en ce que** le composé de la formule III utilisé est 5-(4-tert-butoxy-carbonylpipérazin-1-yl)-2-hydroxybenzaldéhyde.
